# EUROPÄISCHE PATENTANMELDUNG

(11) **EP 4 556 932 A1**
(43) Veröffentlichungstag der Anmeldung: **21.05.2025**
(21) Anmeldenummer: 23210892.8
(22) Anmeldetag: 20.11.2023
(51) Int. Cl.: G01R 33/34, G01R 33/28, A61B 5/00, H01R 12/91, G01R 33/341, G01R 33/36, H01R 13/631

(54) **PATIENTENLAGERUNGSVORRICHTUNG FÜR EINE MAGNETRESONANZVORRICHTUNG**

(71) Anmelder: Siemens Healthineers AG, 91301 Forchheim (DE)
(72) Erfinder: Biber, Stephan, 91056 Erlangen (DE); Matschl, Volker, 96049 Bamberg (DE)
(74) Vertreter: Siemens Healthineers Patent Attorneys

(57) **Zusammenfassung**

Die Erfindung betrifft Patientenlagerungsvorrichtung für eine Magnetresonanzvorrichtung, ein System umfassend einen Patientenlagerungsvorrichtung und zumindest eine Lokalspule und eine Magnetresonanzvorrichtung. Die Patientenlagerungsvorrichtung umfasst einen Patiententisch, der eine Tischoberfläche zur Lagerung eines Patienten aufweist. Der Patiententisch umfasst zumindest ein, insbesondere elektrisches, tischseitiges Kontaktfeld zur elektrischen Kontaktierung zumindest einer Lokalspule. Dabei ist das zumindest eine tischseitige Kontaktfeld bündig in die Tischoberfläche integriert.

## Beschreibung

Die Erfindung betrifft eine Patientenlagerungsvorrichtung für eine Magnetresonanzvorrichtung, ein System umfassend eine Patientenlagerungsvorrichtung und zumindest eine Lokalspule und eine Magnetresonanzvorrichtung.

In der Medizintechnik zeichnet sich die Bildgebung mittels Magnetresonanz (MR), auch Magnetresonanztomographie (MRT, engl. Magnetic Resonance Imaging, MRI) genannt, durch hohe Weichteilkontraste aus. Hierbei werden bei einer Magnetresonanzmessung mit Hilfe einer Magnetresonanzvorrichtung hochfrequente Anregungspulse in einen auf einem Patiententisch gelagerten Patienten ausgesendet, wodurch im Patienten Magnetresonanzsignale ausgelöst werden. Die Magnetresonanzsignale werden von der Magnetresonanzvorrichtung empfangen und zur Rekonstruktion von Magnetresonanzabbildungen verwendet.

Zum Empfang der Magnetresonanzsignale weist die Magnetresonanzvorrichtung oftmals eine oder mehrere Lokalspulen auf, die unmittelbar an den Patienten angeordnet werden. Eine Lokalspule kann wiederum ein oder mehrere Spulenelemente (Antennen) umfassen, mit denen die Magnetresonanzsignale empfangen werden können. Zur Übertragung der empfangenen Magnetresonanzsignale an eine Auswerteeinheit der Magnetresonanzvorrichtung umfasst die Magnetresonanzvorrichtung üblicherweise eine Signalleitung. Die Signalleistung umfasst üblicherweise eine Schnittstelle, beispielsweise in Form einer Steckverbindung, mittels derer die Lokalspule mit der Magnetresonanzvorrichtung für eine Magnetresonanzmessung verbunden werden kann. Nach der Magnetresonanzmessung kann die Verbindung an der Schnittstelle wieder gelöst werden, um die Lokalspule zu entfernen. Diese Schnittstellen sind oftmals direkt am Patiententisch angeordnet.

Um eine Magnetresonanzmessung möglichst hygienisch durchzuführen, ist es notwendig, den Patiententisch, auf dem der Patient während der Magnetresonanzmessung gelagert ist, effektiv reinigen zu können.

Aufgabe der vorliegenden Erfindung ist es insbesondere, einen leicht zu reinigenden Patiententisch vorzuschlagen. Die Aufgabe wird durch die Merkmale der unabhängigen Ansprüche gelöst. Vorteilhafte Ausgestaltungen sind in den abhängigen Ansprüchen beschrieben. Unabhängig vom grammatikalischen Geschlecht eines bestimmten Begriffes sind Personen mit männlicher, weiblicher oder anderer Geschlechteridentität mit umfasst.

Demnach wird eine Patientenlagerungsvorrichtung mit einem Patiententisch für eine Magnetresonanzvorrichtung vorgeschlagen. Der Patiententisch weist eine Tischoberfläche zur Lagerung eines Patienten auf, d.h. der Patient kann insbesondere auf der Tischoberfläche gelagert werden. Der Patiententisch umfasst zumindest eintischseitiges Kontaktfeld zur, insbesondere elektrischen und/oder optischen, Kontaktierung zumindest einer Lokalspule. Dabei ist das zumindest eine tischseitige Kontaktfeld bündig, insbesondere flächig, in die Tischoberfläche integriert.

Insbesondere ist das zumindest eine tischseitige Kontaktfeld plan in die Tischoberfläche integriert. Insbesondere ist das zumindest eine tischseitige Kontaktfeld eben mit Tischoberfläche. Insbesondere weist die Tischoberfläche im Übergang zwischen dem zumindest einen tischseitigen Kontaktfeld und der sich daran anschließenden Tischoberfläche keine Stufe und/oder Erhebung und/oder Absenkung auf. Insbesondere ist das zumindest eine tischseitige Kontaktfeld gegenüber der angrenzenden Tischoberfläche nicht überstehend oder versenkt. Vorzugsweise ist das zumindest eine tischseitige Kontaktfeld selbst glatt und/oder eben. Insbesondere bildet die Oberfläche des zumindest einen tischseitigen Kontaktfeld zusammen mit der angrenzenden Tischoberfläche des Patiententisches eine glatte und/oder flächige und/oder ebene Gesamtoberfläche, insbesondere ohne Vertiefungen und/oder Erhöhungen.

Vorteilhafterweise lässt sich eine solche Tischoberfläche leicht reinigen. Hingegen weisen konventionelle Schnittstellen, insbesondere Steckverbindungen, zur Übertragung von Magnetresonanzsignalen zwischen Lokalspulen und Auswerteeinheiten von Magnetresonanzvorrichtungen, oftmals Öffnungen zur Aufnahme von elektrischen Stiftkontakten auf. In solche Öffnungen können insbesondere Flüssigkeiten eintreten und sind nur schwer zu reinigen.

Vorzugsweise ist das zumindest eine tischseitige Kontaktfeld wasserdicht in die Tischoberfläche integriert. Vorteilhaferweise können so keine Flüssigkeiten in das Innere des Patiententisches eindringen.

Vorzugsweise ist das tischseitige Kontaktfeld geeignet, um darüber Signale und/oder Energie zu übertragen. Vorzugsweise bildet das zumindest eine tischseitige Kontaktfeld einen tischseitigen Teil einer Schnittstelle zur Übertragung von Signalen und/oder Energie. Beispielsweise können über diese Schnittstelle Magnetresonanzsignale übertragen werden, die beispielsweise mittels einer Lokalspule empfangen werden.

Das tischseitige Kontaktfeld ist vorzugsweise ausgebildet, elektrische und/oder optische Signale darüber zu übertragen. Dazu weist es vorzugsweise elektrische und/oder optische Übertragungskanäle auf. Beispielsweise können über optische Übertragungskanäle digitale Signale übertragen werden.

Vorzugsweise umfasst das zumindest eine tischseitige Kontaktfeld zumindest einen elektrischen, insbesondere galvanischen, Kontakt zur Übertragung von Signalen und/oder Energie. Beispielsweise umfasst das zumindest eine tischseitige Kontaktfeld eine Anzahl an elektrischen Kontakten, die einer Anzahl von Empfangskanälen bzw. Spulenelementen einer zu verbindenden Lokalspule entspricht.

Insbesondere weist das zumindest eine tischseitige Kontaktfeld eine plane Oberfläche auf, auf der mehrere elektrische, insbesondere galvanische, Kontakte verteilt sind, beispielsweise in Form einer Matrix. Beispielsweise können die mehrere elektrische, insbesondere galvanische, Kontakte in Reihen und/oder Zeilen auf dem zumindest einen tischseitigen Kontaktfeld angeordnet sein.

Insbesondere ist der zumindest eine elektrische Kontakt zumindest ein galvanischer Kontakt. Vorteilhafterweise weist eine Signalübertragung über galvanische Kontakte nur geringe Verluste auf. Vorzugsweise umfasst der zumindest einen galvanischen Kontakt ein elektrisch leitfähiges Material, wie beispielsweise Kupfer oder Gold. Vorzugsweise ist der zumindest eine galvanische Kontakt ausgebildet, in einem verbundenen Zustand einen korrespondierenden galvanischen Kontakt eines zum tischseitigen Kontaktfeld korrespondieren, insbesondere spulenseitigen, Kontaktfelds (mechanisch) zu berühren. Vorteilhafterweise kann dadurch eine galvantische Verbindung zwischen den Kontakten der Kontaktfelder hergestellt werden.

Vorzugsweise ist der zumindest eine galvanische Kontakt plan in die Oberfläche des tischseitigen Kontaktfelds integriert. Insbesondere weist die Oberfläche des tischseitigen Kontaktfelds im Übergang zum zumindest einen galvanischen Kontakt keine Stufe und/oder Erhebung und/oder Absenkung auf. Insbesondere ist das zumindest eine galvanische Kontakt gegenüber der sonstigen Oberfläche des tischseitigen Kontaktfelds nicht überstehend oder versenkt.

Vorzugsweise ist der das zumindest eine tischseitige Kontaktfeld als Leiterplatte, insbesondere als Leiterkarte, Platine und/oder gedruckte Schaltung (engl. printed circuit board, PCB) ausgebildet. Vorzugsweise ist der zumindest eine, insbesondere galvanische, Kontakt des tischseitigen Kontaktfelds auf einen Träger der Leiterplatte aufgebracht, insbesondere aufgedruckt. Das Material des Trägers ist vorzugsweise ein Verbundwerkstoff, insbesondere ein faserverstärkter Kunststoff, beispielsweise FR-4. Als Kontaktmaterial ist beispielsweise eine Kupferschicht aufgebracht. Vorteilhafterweise lassen sich Leiterplatten besonders einfach herstellen, insbesondere in größerer Stückzahl.

Vorzugsweise ist das zumindest eine tischseitige Kontaktfeld für ein Bedienpersonal der Magnetresonanzvorrichtung frei zugänglich. Insbesondere ist das zumindest eine tischseitige Kontaktfeld für ein Bedienpersonal der Magnetresonanzvorrichtung von zumindest einer Seite, insbesondere von oben, frei zugänglich. Vorteilhafterweise kann es dadurch leicht gereinigt werden. Insbesondere ist das zumindest eine tischseitige Kontaktfeld nicht abgedeckt. Insbesondere ist das tischseitige Kontaktfeld in einem Winkelbereich gegenüber einer Normalen der Oberfläche des tischseitigen Kontaktfelds zugänglich, der mindestens ±30°, insbesondere mindestens ±60, insbesondere ±90° beträgt. Die besagte Normale entspricht dann beispielsweise einer Richtung "direkt von bzw. nach oben".

Insbesondere ist das zumindest eine tischseitige Kontaktfeld so in die Tischoberfläche integriert, dass eine Lokalspule zur Durchführung einer Magnetresonanzmessung daraufgelegt werden kann. Insbesondere kann die Lokalspule formschlüssig auf die Tischoberfläche angeordnet werden.

Vorzugsweise umfasst der Patiententisch zumindest ein Führungselement zur, insbesondere mechanischen, Führung, insbesondere Positionierung, zumindest einer Lokalspule auf dem Patiententisch.

Vorzugsweise umfasst die zumindest eine Lokalspule zumindest ein korrespondierendes Führungselement, das bei einer Positionierung der zumindest einen Lokalspule auf dem Patiententisch mit dem zumindest einen Führungselement des Patiententisches so zusammenwirkt, dass die Lokalspule an eine vorbestimmte Endposition relativ zum Patiententisch gelangt.

Das zumindest eine Führungselement des Patiententisches und das dazu zumindest eine korrespondierende Führungselement der Lokalspule können beispielsweise jeweils eine Führungsfläche aufweisen, entlang derer die Lokalspule in die Endposition gleiten kann.

Vorzugsweise umfasst der Patiententisch zumindest einen Detektor, der ausgebildet ist, eine auf der Tischoberfläche liegende Lokalspule zu detektieren ("Spulen-Anwesenheits-Detektor").

Vorzugsweise ist die Patientenlagerungsvorrichtung ausgebildet ist, bei Detektion einer auf der Tischoberfläche liegenden Lokalspule zumindest eines des zumindest einen tischseitigen Kontaktfelds in einen aktiven Zustand zu schalten.

Vorteilhafterweise kann bei einer Detektion einer auf der Tischoberfläche liegenden Lokalspule zumindest eines des zumindest einen tischseitigen Kontaktfelds aktiviert, insbesondere zugeschaltet und/oder durchgeschaltet, werden. Vorzugsweise ist der aktive Zustand ein Zustand, in dem eine Übertragung von Signalen und/oder Energie über das zumindest eine Kontaktfeld möglich ist.

Vorteilhafterweise wird bei fehlender Detektion einer auf der Tischoberfläche liegenden Lokalspule das entsprechende tischseitige Kontaktfeld deaktiviert, insbesondere werden deren elektrische Kontakte strom- und/oder spannungsfrei geschaltet. Vorzugsweise ist der deaktivierte Zustand ein Zustand, in dem eine Übertragung von Signalen und/oder Energie über das zumindest eine Kontaktfeld nicht (mehr) möglich ist.

Vorteilhafter kann dadurch sichergestellt werden, dass strom- bzw. spannungsführenden Teile, insbesondere Kontakte, nicht in Berührung mit dem Bedienpersonal und/oder den Patienten kommen, beispielsweise beim Einbringen oder Herausnehmen der Lokalspulen. Sobald eine Lokalspule detektiert wird, können die elektrischen Kontakte des Kontaktfeldes elektrisch zugeschaltet werden.

Vorteilhafterweise wird dabei auf eine mechanische Detektion z.B. in Form eines mechanischen Schalters, verzichtet, da ein solcher die Reinigung der Tischoberfläche erschweren würde.

Vorzugsweise umfasst der zumindest eine Detektor zumindest einen kapazitiven Detektor, insbesondere zumindest einen kapazitiven Sensor. Vorzugsweise arbeitet ein kapazitiver Detektor auf Basis einer Änderung einer elektrischen Kapazität eines einzelnen Kondensators oder eines Kondensatorsystems. Vorzugsweise umfasst der zumindest eine kapazitive Detektor eine Elektrode eines solchen Kondensators bzw. Kondensatorsystems. Falls eine Lokalspule auf dem Patiententisch positioniert wird, ändert sich vorteilhafterweise die Kapazität des Kondensators bzw. Kondensatorsystems, so dass die Anwesenheit der Lokalspule erfasst werden kann.

Vorzugsweise umfasst der zumindest eine Detektor zumindest einen induktiven Detektor, insbesondere zumindest einen induktiven Sensor. Vorzugsweise arbeitet ein induktiver Detektor auf Basis einer Änderung einer Induktivität und/oder deren Güte durch eine Lageänderung relativ zu einem leitfähigen und/oder ferromagnetischen Teil.

Vorzugsweise umfasst der zumindest eine Detektor zumindest ein RFID-Lesegerät. Das RFID-Lesegerät ist vorzugsweise ausgebildet, einen RFID-Transponder zu detektieren. Der RFID-Transponder ist insbesondere an oder in der Lokalspule angeordnet. Vorteilhafterweise kann somit auch die Anwesenheit der Lokalspule erkannt werden.

RFID-Lesegeräte erzeugen üblicherweise mithilfe einer Spule und einer Wechselspannung ein bestimmtes elektromagnetisches Feld. Wird ein RFID-Transponder in dieses Feld gebracht, so wird durch das Feld Energie zum Transponder übertragen. Dadurch kann ein Mikrochip des RFID-Transponders aktiviert werden, um vom RFID-Lesegeräte gesendete Befehle zu dekodieren und eine Antwort zu erzeugen, die wiederum vom RFID-Lesegerät erfasst werden kann. Somit kann vorteilhafterweise nicht nur die Anwesenheit einer Lokalspule detektiert werden, sondern es können auch weitere Informationen von der Lokalspule zur Patientenlagerungsvorrichtung übertragen werden, wie beispielsweise eine Seriennummer der Lokalspule.

Das RFID-Lesegeräte kann in dem Patiententisch, aber auch in einem anderen Teil einer Magnetresonanzvorrichtung integriert sein, beispielsweise in einem Tunnel der Magnetresonanzvorrichtung.

Vorzugsweise umfasst der zumindest eine Detektor zumindest einen optischen Detektor. Beispielsweise kann die optische Detektion in einem gewissen Spektralbereich und/oder über eine Glas- oder Kunststofflichtfaser erfolgen. Ferner ist eine Detektion eines Codes, insbesondere eines Strichcodes oder eines QR-Codes, denkbar, der an der zu detektierenden Lokalspule angeordnet ist.

Vorzugsweise ist der zumindest eine Detektor ausgebildet, Eigenschaften, insbesondere Resonanzeigenschaften, von Spulenelementen einer Lokalspule zu erfassen, wenn die Lokalspule auf der Tischoberfläche liegt. Beispielsweise könnte der zumindest eine Detektor eine Resonanzschleife umfassen. Mittels einer Resonanzüberhöhung und/oder Verkopplung mit einer in einer Lokalspule integrierten weiteren Resonanzschleife könnte dann die Anwesenheit der Lokalspule detektiert werden.

Vorzugsweise ist der zumindest eine Detektor unter der Tischoberfläche angeordnet ist. Vorteilhafterweise kann dadurch der Detektor vor möglichen Beschädigungen besser geschützt werden.

Beispielsweise kann das zumindest eine tischseitige Kontaktfeld als Leiterplatte ausgebildet sein, wobei auf der Oberfläche der Leiterplatte die elektrischen, insbesondere galvanischen Kontakte angeordnet sind; in tieferliegenden Schichten dieser Leiterplatte können hingegen induktive und/oder kapazitive Detektoren eingebaut werden, so dass sie, insbesondere durch das Bedienpersonal, nicht berührbar sind.

Ferner wird ein System vorgeschlagen, das eine vorangehend beschriebene Patientenlagerungsvorrichtung und zumindest eine Lokalspule umfasst. Dabei umfasst die zumindest eine Lokalspule zumindest ein spulenseitiges Kontaktfeld, das mit dem zumindest einen tischseitigen Kontaktfeld korrespondiert. Die Lokalspule kann insbesondere eine Sendespule zum Senden von HF-Signalen, eine Empfangsspule zum Empfangen von Magnetresonanzsignalen oder eine Sende-Empfangsspule zum Senden von HF-Signalen und Empfangen von Magnetresonanzsignalen sein.

Insbesondere weisen das zumindest eine spulenseitige Kontaktfeld und das zumindest eine tischseitige Kontaktfeld jeweils eine Kontaktanordnung auf, die bei Positionierung der Lokalspule auf dem Patiententisch in Deckung gebracht wird, so dass sich die Kontakte berühren und eine elektrische und/oder optische Verbindung hergestellt wird.

Vorzugsweise umfasst das zumindest eine spulenseitige Kontaktfeld und/oder das tischseitige Kontaktfeld zumindest einen elektrischen Federkontakt und/oder elektrischen Schleifkontakt. Diese eigenen sich vorteilhafterweise besonders gut, um eine zuverlässige Kontaktierung herzustellen. Ferner sind Federkontakte und/oder Schleifkontakte vorteilhafterweise selbstreinigend.

Vorzugsweise umfasst die zumindest eine Lokalspule einen starren Bereich, in dem das spulenseitige Kontaktfeld angeordnet ist. Dieser starre Bereich kann beispielsweise eine Unterseite einer Kopfspule oder einer Wirbelsäulenspule sein. Vorzugsweise weist die Lokalspule kein Kabel zur Kontaktierung der Lokalspule auf. Vorteilhafterweise kann mittels der Kontaktfelder eine kabellose Direktsteckung der Lokalspule an den Patiententisch durchgeführt werden. Dies vereinfacht vorteilhafterweise den Arbeitsablauf einer Magnetresonanzuntersuchung, weil die Positionierung der Lokalspule auf dem Patiententisch und die Kontaktierung der Lokalspule gleichzeitig erfolgen kann.

Vorteilhafterweise werden die Kontakte des spulenseitige Kontaktfelds gegen mechanische Beschädigungen geschützt. Vorzugsweise umfasst die Lokalspule einen Schutzmechanismus, der das spulenseitige Kontaktfeld automatisch beim Positionieren der Lokalspule auf dem Patiententisch freilegt. Dies kann insbesondere mechanisch erfolgen, beispielsweise mittels einer gefederten Schutzumrandung, die um das spulenseitige Kontaktfeld herum angeordnet ist; die Schutzumrandung kann vorzugsweise in das Innere der Lokalspule eingedrückt werden, wenn die Lokalspule auf den Patiententisch gelegt wird. Ferner ist denkbar, dass das spulenseitige Kontaktfeld erst aus der Spule ausfährt, wenn ein Pin beim Auflegen der Lokalspule auf den Tisch eingedrückt wird.

Ferner wird eine Magnetresonanzvorrichtung mit einer vorangehend beschriebenen Patientenlagerungsvorrichtung vorgeschlagen.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: eine Magnetresonanzvorrichtung mit einer Patientenlagerungsvorrichtung und zwei Lokalspulen, nämlich einer Kopfspule und einer Wirbelsäulenspule,
- Fig. 2: eine Patientenlagerungsvorrichtung mit zwei Lokalspulen in einem nicht-verbundenen Zustand,
- Fig. 3: eine Patientenlagerungsvorrichtung mit zwei Lokalspulen in einem verbundenen Zustand,
- Fig. 4: Draufsicht auf eine Oberfläche der Patientenlagerungsvorrichtung.

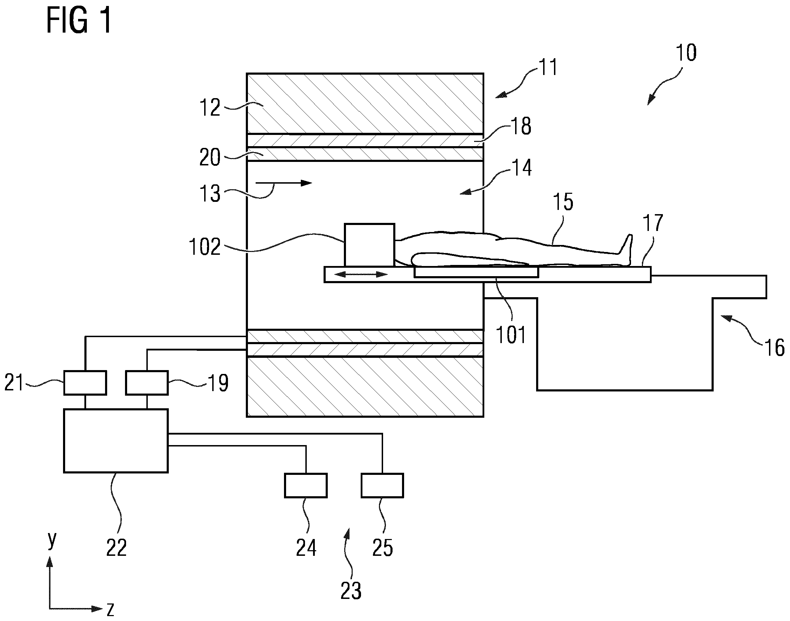

In Fig. 1 ist eine Magnetresonanzvorrichtung 10 schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds 13 aufweist. Zudem umfasst die Magnetresonanzvorrichtung 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 in z-Richtung bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in einen Untersuchungsraum ein, der im Wesentlichen von einem Patientenaufnahmebereich 14 der Magnetresonanzvorrichtung 10 gebildet ist. Dadurch stellt sich dem von dem Hauptmagneten 12 erzeugten Hauptmagnetfeld 13 eine Anregung von Atomkernen ein. Durch Relaxation der angeregten Atomkerne werden Magnetresonanzsignale erzeugt. Die Magnetresonanzsignale werden von Lokalspulen empfangen, die hier als Wirbelsäulenspule 101 und als Kopfspule 102 ausgebildet sind.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung der Magnetresonanzsignale, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzabbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

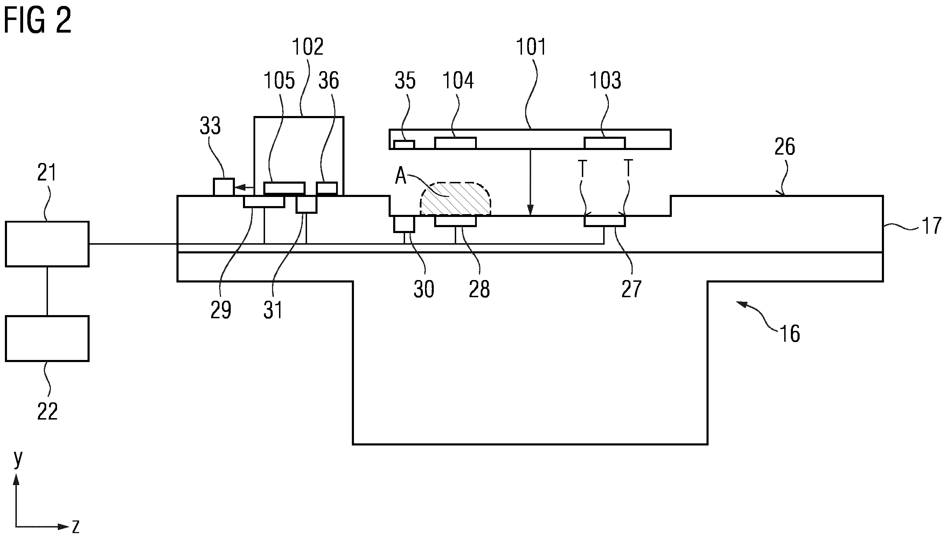

In Fig. 2 ist eine Patientenlagerungsvorrichtung 16 mit einem Patiententisch 17 zum Einsatz in einer Magnetresonanzvorrichtung 10 dargestellt. Der Patiententisch weist eine Tischoberfläche 26 auf, auf der insbesondere Lokalspulen 101, 102 und/oder der Patient 15 angeordnet werden können. Der Patiententisch 17 umfasst zwei tischseitige Kontaktfelder 27, 28 zur elektrischen Kontaktierung einer Wirbelsäulenspule 101 und ein tischseitiges Kontaktfeld 29 zur elektrischen Kontaktierung einer Kopfspule 102. Die tischseitigen Kontaktfelder 27, 28, 29 sind bündig, insbesondere wasserdicht, in die Tischoberfläche integriert. Die tischseitigen Kontaktfelder 27, 28, 29 bilden mit der angrenzenden Tischoberfläche 26 eine glatte Gesamtoberfläche. Insbesondere ist keine Kante o.ä. im Übergang zu den Kontaktfeldern vorhanden, insbesondere an den Übergangsstellen T, die beispielhaft für das tischseitige Kontaktfeld 27 eingezeichnet sind. Vorteilhafterweise lassen sich so die Kontaktfelder 27, 28, 29 besonders einfach reinigen.

Die Kontaktfelder 27, 28, 29 sind über die Hochfrequenzantennensteuereinheit 21 mit der Systemsteuereinheit 22 der Magnetresonanzvorrichtung 10 verbunden. Dadurch können Signale und/oder Informationen von der Systemsteuereinheit 22 zu den Kontaktfeldern 27, 28, 29 und/oder von den Kontaktfeldern 27, 28, 29 zur Systemsteuereinheit 22 übertragen werden.

Die tischseitigen Kontaktfelder 27, 28, 29 sind frei zugänglich, wenn die Lokalspulen 101, 102 nicht auf dem Patiententisch angeordnet sind. Insbesondere kann das Bedienpersonal von oben leicht die Kontaktfeldern 27, 28, 29 erreichen. Der Zugang zu den tischseitigen Kontaktfeldern 27, 28, 29 ist nicht durch etwaige Abdeckungen erschwert. Beispielhaft ist für das tischseitige Kontaktfelder 28 ein frei zugänglicher Bereich A eingezeichnet, in dem sich keine die Zugänglichkeit beschränkenden Elemente der Magnetresonanzvorrichtung 10 befinden.

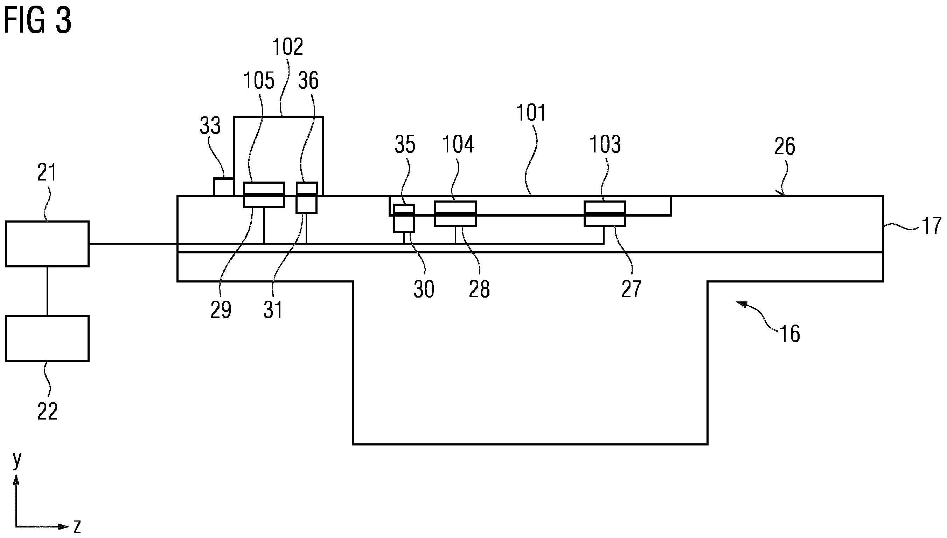

Die Tischoberfläche 26 ist zur Aufnahme der Wirbelspulenspule 101 ausgeformt. Dazu weist die Tischoberfläche 26 eine Vertiefung auf, die durch die Kante 34 begrenzt wird. Diese Kante 34 fungiert hier auch als Führungselement, um die Wirbelsäulenspule 101 durch Bewegung in y-Richtung in die in Fig. 3 gezeigt Endposition zu bringen. In dieser Endposition berühren sich die tischseitigen Kontaktfelder 27, 28 und die spulenseitigen Kontaktfelder 103, 104, d.h. das tischseitige Kontaktfeld 27 kommt in Kontakt mit dem spulenseitigen Kontaktfeld 103, und das tischseitige Kontaktfeld 28 kommt in Kontakt mit dem spulenseitigen Kontaktfeld 104. Insbesondere weisen sowohl die tischseitigen Kontaktfelder 27, 28 also auch die spulenseitigen Kontaktfelder 103, 104 jeweils mehrere elektrische, insbesondere galvanische Kontakte auf, die sich im verbundenen Zustand berühren und so Signale und/oder Energie über diese Schnittstelle übertragen können.

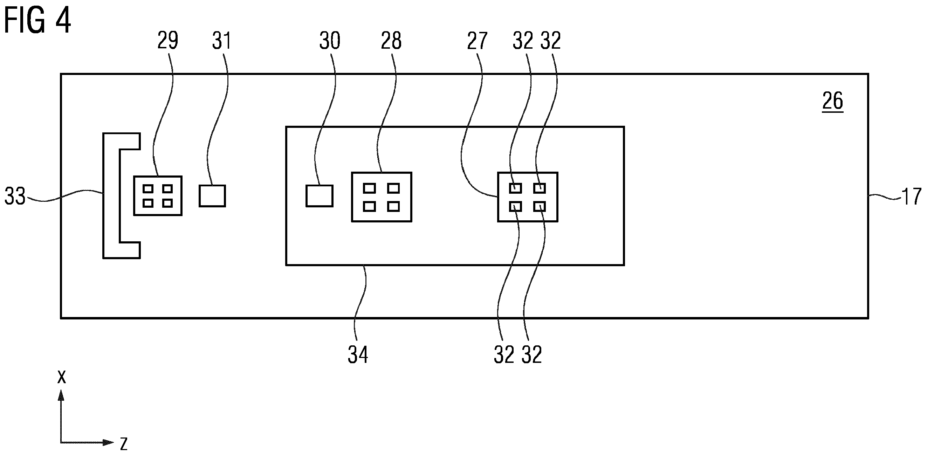

Ebenso kann die Kopfspule 102 in diesem Beispiel durch eine Bewegung in z-Richtung in die in Fig. 3 dargestellte Endposition gelangen. Dabei wird die Bewegung durch das Führungselement 33 unterstützt, das in Fig. 4 in der Draufsicht gezeigt wird. In der Endposition kommt das tischseitige Kontaktfeld 29 mit dem spulenseitigen Kontaktfeld 105 der Kopfspule in Verbindung.

In der Draufsicht sind auch elektrische, insbesondere galvanische, Kontakte 32 der tischseitigen Kontaktfelder 27, 28, 29 dargestellt (aus Gründen der Übersichtlichkeit nur für das Kontaktfeld 27 mit Bezugszeichen versehen). Es ist aber auch denkbar, eine oder mehrere optische Schnittstellen zur Übertragung von optischen Signalen auf den Kontaktfeldern anzuordnen. Wenn die Lokalspulen in ihre Endpositionen kommen, kann durch diese Kontakte 32 eine elektrische, insbesondere galvanische, Verbindung mit dazu korrespondierenden Kontakten der spulenseitigen Kontaktfelder 103, 104, 105 hergestellt werden, indem sich diese Kontakte berühren. Im dargestellten Beispiel umfasst jedes Kontaktfeld vier auch elektrische, insbesondere galvanische, Kontakte 32. Auf einem Kontaktfeld können aber auch mehr oder weniger elektrische, insbesondere galvanische, Kontakte angeordnet sein. Ferner können die Kontaktfelder auch eine unterschiedliche Anzahl an elektrische, insbesondere galvanische, Kontakten aufweisen.

Die Kontakte 32 der tischseitigen Kontaktfelder 27, 28, 29 und/oder die Kontakte der spulenseitigen Kontaktfelder 103, 104, 105 können insbesondere als Federkontakte und/oder Schleifkontakte ausgeführt werden.

Vorzugsweise befinden sich die Kontakte der spulenseitigen Kontaktfelder 103, 104, 105 auf der Unterseite der Lokalspulen 101, 102. Vorteilhafterweise liegen die Kontakte der spulenseitigen Kontaktfelder 103, 104, 105 erst frei, wenn die Lokalspule 101, 102 auf die plane Oberfläche des Patiententisches 17 gestellt bzw. gelegt wird. Dadurch können die Kontakte gegen vorteilhafterweise gegen mechanische Beschädigungen geschützt werden.

Beispielsweise umfasst die Lokalspule 101, 102 auf der Unterseite eine gefederte Schutzumrandung um das spulenseitige Kontaktfeld 103, 104, 105, die sich zurückzieht bzw. eingedrückt wird, wenn die Lokalspule 101, 102 auf die Oberfläche 26 des Patiententisches 17 gelegt wird. Es ist auch denkbar, dass die Lokalspule 101, 102 auf der Unterseite einen Stift aufweist, der beim Auflegen der Lokalspule 101, 102 auf die Oberfläche 26 des Patiententisches 17 eingedrückt wird und mittels eines geeigneten mechanischen Mechanismus bewirkt, dass die spulenseitigen Kontaktfelder 103, 104, 105 und/oder deren Kontakte ausfahren, so dass sie dann freiliegen.

Ferner sind in dem Patiententisch 17, insbesondere unter der Tischoberfläche 26, Detektoren 30, 31 angeordnet, die ausgebildet sind zu detektieren, ob eine jeweils zugehörige Lokalspule in einer vorbestimmten Position, insbesondere in den voran beschriebenen Endpositionen, auf dem Patiententisch 17 liegt bzw. angeordnet sind. Mit dem Detektor 30 kann etwa hier die Anwesenheit der Wirbelsäulenspule 101 erkannt werden und mit dem Detektor 31 die Anwesenheit der Kopfspule 102.

Solange keine Anwesenheit der Wirbelsäulenspule 101 bzw. der Kopfspule 102 detektiert wird, befinden sich die jeweiligen tischseitigen Kontaktfelder 27, 28, 29 in einem inaktiven Zustand. So ein inaktiver Zustand ist vorzugsweise dadurch charakterisiert, dass die elektrischen Kontakte der tischseitigen Kontaktfelder 27, 28, 29 strom- und/oder spannungsfrei sind. Bei Detektion einer auf der Tischoberfläche 26 liegenden Lokalspule 101, 102 wird das zugehörige tischseitigen Kontaktfeld 27, 28, 29 in einen aktiven Zustand zu geschaltet. Wird also beispielsweise durch den Detektor 30 erkannt, dass die Wirbelsäulenspule 101 auf die Tischoberfläche 26 gelegt wurde, werden die Kontaktfelder 27, 28 in den aktiven Zustand geschaltet. Entsprechend kann mittels des Detektors 31 das tischseitige Kontaktfeld 29 in den aktiven oder inaktiven Zustand geschaltet werden.

Um eine solche Umschaltung durchzuführen, kann beispielsweise ein entsprechendes Detektionssignal vom Detektor 30, 31 an die Systemsteuereinheit 22 gesendet werden, welche wiederum eine Schaltung veranlasst.

Beispielsweise basiert der Detektor 30, 31 auf einer kapazitiven Detektion. Dazu umfasst der Detektor beispielsweise eine Elektrode, die mit einem Gegenstück 35, 36 der Lokalspule 101, 102 kapazitiv zusammenwirkt. Eine durch Anordnung der Lokalspule 101, 102 auf dem Patiententisch 101, 102 kann eine Kapazitätsänderung hervorgerufen werden, die als Detektorsignal an die Systemsteuereinheit 22 übertragen wird.

Gemäß einer anderen Ausführungsform kann der Detektor 30, 31 auch durch eine induktive Abkopplung die Anwesenheit der Lokalspule 101, 102 auf dem Patiententisch 17 erkennen.

Die tischseitigen Kontaktfelder 27, 28, 29, können insbesondere in Form einer Leiterplatte aufgebaut sein. Induktive und/oder kapazitive Detektorelemente, insbesondere Kontakte, könnten insbesondere direkt in die Leiterplatte eingebaut werden, die auch die galvanischen Kontakte 32 enthalten. Sie würden dann vorzugsweise in tieferliegenden Schichten dieser Leiterplatte liegen, so dass sie nicht direkt vom Bedienpersonal berührbar sind. Von Vorteil wäre die einfache Integration dieses "Spulen-Anwesenheits-Detektors" in die Leiterplatte, die auch die galvanischen Kontakte, die dann zugeschaltet werden, enthält.

Eine weitere Möglichkeit ist eine Detektion eines RFID-Transponders, der in oder an der Lokalspule 101, 102 angebracht ist. Dazu kann der Detektor 30, 31 ein RFID-Lesegerät umfassen. Ein solches RFID-Lesegerät könnte allerdings an einer anderen Stelle der Magnetresonanzvorrichtung 10 angeordnet sein, wie z.B. an der Begrenzung des Patientenaufnahmebereichs 14 bzw. am Gehäuse des Tunnels.

Gemäß einer anderen Ausführungsform umfasst der Detektor 30, 31 jeweils einen optischen Scanner, der ausgebildet ist, eine optische Markierung, z.B. einen Strichcode und/oder einen QR-Code, zu erkennen, der an der Lokalspule 101, 102 angebracht ist. Die optische Detektion ist auch Detektion z.B. in einem gewissen Spektralbereich, etwa über eine Glas- oder Kunststofflichtfaser möglich.

Ferner ist denkbar, dass wobei der Detektor 30, 31 ausgebildet ist, Eigenschaften, insbesondere Resonanzeigenschaften, von Spulenelementen der Lokalspule 101, 102 zu erfassen, wenn die Lokalspule 101, 102 auf der Tischoberfläche 26 liegt. Beispielsweise könnte in der Lokalspule 101, 102 ein einfacher Resonanzloop angeordnet sein, der mit einem Resonanzloop in dem Detektor 30, 31 im Patiententisch 17 koppelt, so dass eine Resonanzüberhöhung das Detektionssignal bewirkt.

Es wird abschließend noch einmal darauf hingewiesen, dass es sich bei der vorhergehend detailliert beschriebenen Patientenlagerungsvorrichtung und Magnetresonanzvorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. Patientenlagerungsvorrichtung (16) mit einem Patiententisch (17) für eine Magnetresonanzvorrichtung (10),
wobei der Patiententisch (17) eine Tischoberfläche (26) zur Lagerung eines Patienten aufweist,
wobei der Patiententisch (17) zumindest ein tischseitiges Kontaktfeld (27, 28, 29) zur Kontaktierung zumindest einer Lokalspule (101, 102) aufweist,
wobei das zumindest eine tischseitige Kontaktfeld (27, 28, 29) bündig in die Tischoberfläche (26) integriert ist.

2. Patientenlagerungsvorrichtung (16) nach Anspruch 1,
wobei das zumindest eine tischseitige Kontaktfeld (27, 28, 29) wasserdicht in die Tischoberfläche (26) integriert ist.

3. Patientenlagerungsvorrichtung (16) nach einem der vorangehenden Ansprüche,
wobei das zumindest eine tischseitige Kontaktfeld (27, 28, 29) zumindest einen galvanischen Kontakt (32) und/oder zumindest eine optische Schnittstelle umfasst.

4. Patientenlagerungsvorrichtung (16) nach einem der vorangehenden Ansprüche,
wobei das zumindest eine tischseitige Kontaktfeld (27, 28, 29) als Leiterplatte, insbesondere als Leiterkarte, Platine und/oder gedruckte Schaltung, ausgebildet ist.

5. Patientenlagerungsvorrichtung (16) nach einem der vorangehenden Ansprüche,
wobei das zumindest eine tischseitige Kontaktfeld (27, 28, 29) frei zugänglich ist.

6. Patientenlagerungsvorrichtung (16) nach einem der vorangehenden Ansprüche,
wobei der Patiententisch (17) zumindest ein Führungselement (33, 34) zur Führung, insbesondere Positionierung, zumindest einer Lokalspule (101, 102) umfasst.

7. Patientenlagerungsvorrichtung (16) nach einem der vorangehenden Ansprüche,
wobei der Patiententisch (17) zumindest einen Detektor (30, 31) umfasst, der ausgebildet ist, eine auf der Tischoberfläche (26) liegende Lokalspule (101, 102) zu detektieren.

8. Patientenlagerungsvorrichtung (16) nach Anspruch 7, wobei die Patientenlagerungsvorrichtung (16) ausgebildet ist, bei Detektion einer auf der Tischoberfläche (26) liegenden Lokalspule (101, 102) zumindest eines des zumindest einen tischseitigen Kontaktfelds (27, 28, 29) in einen aktiven Zustand zu schalten.

9. Patientenlagerungsvorrichtung (16) nach einem der Ansprüche 7 oder 8,
wobei der zumindest eine Detektor (30, 31) zumindest einen kapazitiven Detektor und/oder zumindest einen induktiven Detektor und/oder zumindest ein RFID-Lesegerät umfasst.

10. Patientenlagerungsvorrichtung (16) nach einem der Ansprüche 7 bis 9,
wobei der zumindest eine Detektor (30, 31) einen optischen Detektor umfasst.

11. Patientenlagerungsvorrichtung (16) nach einem der Ansprüche 7 bis 10,
wobei der zumindest eine Detektor (30, 31) ausgebildet ist, Eigenschaften, insbesondere Resonanzeigenschaften, von Spulenelementen einer Lokalspule (101, 102) zu erfassen, wenn die Lokalspule (101, 102) zu auf der Tischoberfläche (26) liegt.

12. Patientenlagerungsvorrichtung (16) nach einem der Ansprüche 7 bis 11,
wobei der zumindest eine Detektor (30, 31) unter der Tischoberfläche (26) angeordnet ist.

13. System umfassend einen Patientenlagerungsvorrichtung (16) nach einem der vorangehenden Ansprüche und zumindest eine Lokalspule (101, 102), insbesondere eine Kopfspule (102) und/oder eine Wirbelsäulenspule (101),
wobei die zumindest eine Lokalspule (101, 102) zumindest ein spulenseitiges Kontaktfeld (103, 104, 105) umfasst, das mit einem tischseitigem Kontaktfeld (27, 28, 29) korrespondiert.

14. System nach Anspruch 13,
wobei das zumindest eine spulenseitige Kontaktfeld (103, 104, 105) und/oder das tischseitige Kontaktfeld (27, 28, 29) zumindest einen Federkontakt und/oder Schleifkontakt umfasst.

15. Magnetresonanzvorrichtung mit einer Patientenlagerungsvorrichtung (16) nach einem der Ansprüche 1 bis 12.
